(19) Europäisches Patentamt
European Patent Office
Office européen des brevets

(11) Veröffentlichungsnummer: **0 206 155**
**B1**

# (12) EUROPÄISCHE PATENTSCHRIFT

(45) Veröffentlichungstag der Patentschrift: **18.10.89**

(51) Int. Cl.⁴: **C 07 D 471/22**, A 61 K 31/435 // (C07D471/22, 221:00, 221:00, 221:00, 221:00)

(21) Anmeldenummer: **86108054.7**

(22) Anmeldetag: **12.06.86**

## (54) Neue aromatische Verbindungen, ihre Herstellung und Verwendung.

(30) Priorität: **22.06.85 DE 3522475**

(43) Veröffentlichungstag der Anmeldung: **30.12.86 Patentblatt 86/52**

(45) Bekanntmachung des Hinweises auf die Patenterteilung: **18.10.89 Patentblatt 89/42**

(84) Benannte Vertragsstaaten: **AT BE CH DE FR GB IT LI LU NL SE**

(56) Entgegenhaltungen:
**DE-A- 2 360 475**

**Chemical Abstracts, Band 51, Nr. 11, 10. Juni 1957, Columbus, Ohio, USA M.RINK "17-Hydroxysparteine", Spalte 8114, Zusammenfassung-Nr. 8 114d**
**Chemical Abstracts, Band 67, Nr. 5, 31 Juli 1967, Columbus, Ohio, USA E:NOWACKI "Conversion of oxosparteins into phenylsparteins and isolation of carbon atom from the carbonyl group" Spalte 1, Zusammenfassung-Nr. 22 070h**

(73) Patentinhaber: **Kali-Chemie Pharma GmbH, Hans-Böckler-Allee 20 Postfach 220, D-3000 Hannover 1 (DE)**

(72) Erfinder: **Schön, Uwe, Dr. rer. nat., Föhrenkamp 12, D-3167 Burgdorf (DE)**
Erfinder: **Kehrbach, Wolfgang, Dr. rer. nat., Altenbekener Damm 41, D-3000 Hannover 1 (DE)**
Erfinder: **Hachmeister, Bernd, Dr. rer. nat., Möwenkamp 4, D-3004 Isernhagen 1 (DE)**
Erfinder: **Buschmann, Gerd, Dr. med. vet., Ernst-Ebeling-Strasse 9, D-3000 Hannover 72 (DE)**
Erfinder: **Kühl, Ulrich Gottfried, Dr. med. vet., Franzburger Strasse 10, D-3007 Gehrden 1 (DE)**

(74) Vertreter: **Lauer, Dieter, Dr., c/o Kali-Chemie AG Postfach 220 Hans-Böckler-Allee 20, D-3000 Hannover 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 206 155 B1

ACTORUM AG

**Beschreibung**

Die Erfindung betrifft neue aromatische Verbindungen der Spartein-Reihe, sowie deren Verwendung und ein Verfahren zu ihrer Herstellung. Sie umfaßt ferner Arzneimittel, die solche Verbindungen enthalten, sowie Verfahren zur Herstellung solcher Arzneimittel.

Vom Spartein, einem aus Besenginster gewinnbaren Alkaloid, sind herzwirksame Eigenschaften, insbesondere sein Einfluß auf den Herzrhythmus, bereits eingehend beschrieben.

Eine Wirkungssteigerung hinsichtlich der Verlängerung der Refraktärzeit konnte mit in 17-Stellung durch Alkyl substituierte Sparteine erreicht werden, also Verbindungen, die nachfolgende Struktur aufweisen.

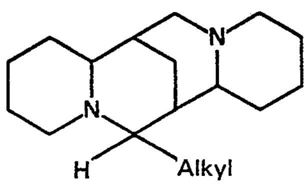

Solche Verbindungen sind aus der DE-OS 2 360 475 bekannt. Ferner ist aus der EP-A1-0 046 565 ein dimeres Spartein mit antiarrythmischer Wirkung, das 17,17'-Bisspartein bekannt.

Die Verbindungen nach dem Stand der Technik zeigen zwar gute Eigenschaften bei der Behandlung von Herzrhythmus-Beschwerden, bezüglich des Wirkungsprofiles sind sie aber noch verbesserungsfähig.

Aufgabe der Erfindung ist es, neue Sparteinderivate mit abgeändertem Wirkungsprofil zur Verfügung zu stellen.

Die Aufgabe wird gelöst durch die Bereitstellung der in den Patentansprüchen angegebenen Verbindungen.

Die Erfindung umfaßt neue aromatische verbindungen der allgemeinen Formel (I)

$$S\text{-}(CH_2)_n\text{-}A \qquad (I)$$

in der
S den 17-Spartein-Rest
n 0 oder 1
und A

a1) 2-Furyl, 2-Thienyl, 2-(N-$C_1$-$C_4$-Alkyl)-pyrryl für n = 0 oder
a2) 3-Furyl, 3-Thienyl für n = 1 oder
b) Pyridyl, oder
c) substituiertes Phenyl der Formel (II)

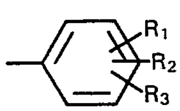 (II)

bedeutet, in dem unabhängig voneinander einer oder zwei der Reste $R_1$ bis $R_3$ Wasserstoff und einer oder mehrere der Reste $R_1$ bis $R_3$ unabhängig voneinander

C1) Alkyl mit 1-4 Kohlenstoffatomen,
C2) Alkoxy mit 1-4 Kohlenstoffatomen,
C3) Fluor,
C4) Chlor,
C5) Brom,
C6) Trifluormethyl,
C7) zwei benachbarte Reste gemeinsam Alkylendioxy mit 1-2 Kohlenstoffatomen,
C8) $-CH-O-(CH_2)_2-O,$
C9) Hydroxy,
C10) -Co-$R_4$

sind, wobei $R_4$ Wasserstoff, Alkoxy mit 1-4 Kohlenstoffatomen, Hydroxy oder gegebenenfalls durch Alkylgruppen mit 1-4 Kohlenstoffatomen substituiertes Amino ist, sowie deren pharmakologisch verwendbare Säureadditionssalze.

Sofern A Pyridyl bedeutet, ist darunter 2-, 3- oder 4-Pyridyl, vorzugsweise 2- oder 4-Pyridyl zu verstehen.

Als Alkyl kommt erfindungsgemäß sowohl verzweigtes als auch unverzweigtes Alkyl in Frage. Als Alkyl wird im folgenden Alkyl mit bis zu vier C-Atomen verstanden. Bevorzugte Alkylreste sind somit als verzweigter Rest der Isopropyl-, sec.-Butyl-, (2-Methylpropyl)-Rest und als unverzweigtes Alkyl der Methyl-, Äthyl-, n-Propyl-, n-Butyl-Rest.

Unter Alkoxy-Rest wird Alkoxy mit bis zu vier C-Atomen wie Methoxy, Äthoxy, Propoxy verstanden.

Alkylendioxy bezeichnet insbesondere die Gruppierung $-O-(CH_2)_q-O-$ mit $q = 1$ bis 2.

Entsprechend einer Variante der Erfindung bedeutet keiner der Reste $R_1$ bis $R_3$ Wasserstoff. Die entsprechenden Verbindungen werden im folgenden als trisubstituiert bezeichnet. Ein Beispiel für solche Verbindungen ist für n = 1 die Substitution 3,4,5-trimethoxy.

Eine andere Variante der Erfindung sieht vor, daß einer der Reste $R_1$ bis $R_3$ Wasserstoff ist. Solche Verbindungen werden als disubstituiert bezeichnet. Beispiele für solche Verbindungen sind für n = 1 die Substitutionen 2,4-Dimethyl, 2,3-Dimethoxy, 2,4--Dichlor, 2,6-Dichlor, 3,5-Dichlor, 2-Fluor-3-methyl, 2,6-Difluor, 2-Fluor-6-chlor, 3-Methoxy-4-formyl.

Ein besonderer Fall der Disubstitution ist, daß zwei benachbarte Reste $R_1$ bis $R_3$ gemeinsam eine Alkylendioxygruppe bilden. Beispiele für solche Verbindungen sind für n = 1 die Substituenten 3,4-Methylendioxy und 3,4-Äthylendioxy.

In einer Reihe von Verbindungen besitzen zwei der Reste $R_1$ bis $R_3$ die Bedeutung Wasserstoff. Die entsprechenden Verbindungen werden im folgenden als monosubstituiert bezeichnet. Der Substituent kann dabei in ortho-, meta- oder para-Stellung zur Verknupfung mit dem Sparteinrest stehen. Für n = 0 in Formel (I) sind monosubstituierte Verbindungen der Formel (Vm)

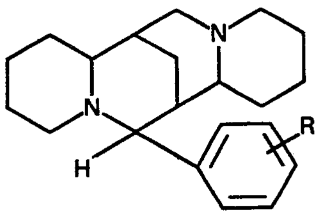

(Vm)

besonders zu nennen, in denen R die folgende Bedeutung besitzt: 3-Methyl, 2-Methoxy, 3-Methoxy, 4-Methoxy, 4-Hydroxy-, 2-Trifluormethyl, 3-Trifluormethyl, 4-Trifluormethyl, 3-Fluor, 4-Fluor, 3-Chlor, 4-Chlor, 4-Brom, 3-Formyl.

Für n = 1 in Formel (I) sind monosubstituierte Verbindungen der Formel (VIm)

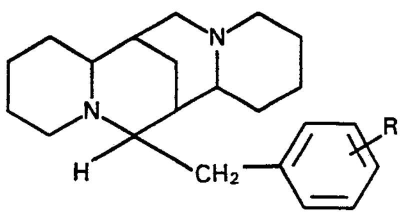

(VIm)

besonders zu nennen, in denen R die folgende Bedeutung besitzt.

2-Methyl, 3-Methyl, 4-Methyl, 2-Methoxy, 3--Methoxy, 4-Methoxy, 3-Hydroxy, 2-Fluor, 3-Fluor, 4-Fluor, 2-Chlor, 3-Chlor, 4-Chlor, 2-Brom, 3-Brom, 4-Brom, 3-Trifluormethyl, 4-Formyl, 4-(N,N-Diisopropyl)-aminocarbonyl.

Besonders bevorzugt sind solche Erfindungen der Formel (I), in denen n die Bedeutung 1 besitzt.

Die Erfindung umfaßt auch Arzneimittel, die mindestens eine der vorgenannten Verbindungen gemäß Formel (I) oder deren pharmakologisch verwendbares Additionssalz enthalten.

Als pharmakologisch verwendbaren Säureadditionssalze eignen sich beispielsweise wasserlösliche und wasserunlösliche Salze von anorganischen oder organischen Säuren wie z.B. das Hydrochlorid, Hydrobromid, Hydrojodid, Phosphat, Nitrat, Sulfat, Perchlorat, Acetat, Citrat, Gluconat, Benzoat, Propionat, Butyrat, Salicylat, Sulfosalicylat, Maleinat, Laurat, Fumarat, Succinat, Oxalat, Tartrat, Stearat, Tosylat (p-Toluolsulfonat), 2-Hydroxy-3-naphthoat, 3-Hydroxy-2-naphthoat, Mesylat (Methansulfonat), Naphthalinsulfonat.

Die Erfindung umfaßt ferner Arzneimittel, die als Wirkstoff mindestens eine Verbindung der allgemeinen Formel (I) enthalten, in der A zusätzlich unsubstituiertes Phenyl bedeutet, oder deren pharmakologisch verwendbare Additionssalze.

Diese letztgenannten Verbindungen sind an sich bereits bekannt (Rink, Grabowski, Archiv d. Pharmazie *289*, 1956, S. 702). Über ihre pharmakologischen Eigenschaften ist allerdings noch nichts beschrieben. Ihre Verwendung bei der therapeutischen Behandlung ist daher neu. Ebenso sind auch Arzneimittel neu, die diese an sich bekannten Verbindungen enthalten.

Die Erfindung betrifft auch ein Verfahren zur Herstellung von Arzneimitteln, die mindestens eine Verbindung der Formel (I) oder Verbindungen der Formel (I), in denen A zusätzlich unsubstituiertes Phenyl bedeutet, oder deren pharmakologisch verwendbare Säureadditionssalze enthalten. Dieses verfahren besteht darin, die genannten Verbindungen mit inerten, pharmakologisch geeigneten Trägerstoffen zu vermischen und in an sich bekannter Weise in galenische Zubereitungen zu überführen. Solche Zubereitungen können z.B. sein Tabletten, Dragees, Kapseln, Pulver, Granulate, wäßrige und ölige Suspensionen, Emulsionen, Sirupe oder Lösungen für die orale Anwendung, Suppositorien für die rektale Anwendung oder steril injizierbare Suspensionen oder Lösungen für die parenterale Applikation.

Die Erfindung umfaßt auch Verfahren zur Herstellung von Verbindungen der Formel (I).

In einer Variante i) wird dabei 17-Hydroxyspartein oder 17-Dehydrospartein-Salz, insbesondere das Perchlorat, mit einer Grignard-Verbindung der Formel (III)

$$A\text{-}(CH_2)_n\text{-}MgHal \qquad (III)$$

umgesetzt, wobei n 0 oder 1 ist, A die unter a1) a2), b), c1) bis c8) angegebene Bedeutung hat und Hal Halogen, insbesondere Brom oder Chlor bedeutet, zu den entsprechend substituierten Verbindungen der Formel (I).

In einer variante ii) wird 17-Dehydrospartein-Salz, insbesondere des Perchlorat, mit einer metallorganischen Verbindung der Formel (IV)

$$A\text{-}(CH_2)_n\text{-}Li \qquad (IV)$$

umgesetzt, wobei n 0 oder 1 ist und A die unter a1), b), c1) bis c8) angegebene Bedeutung hat zu den entsprechend substituierten Verbindungen der Formel (I).

Die Herstellung der Verbindungen (III) bzw. (IV) ist an sich bekannt und erfolgt durch Umsetzung von Verbindungen der Formel (VII)

$$A\text{-}(CH_2)_n\text{-}Hal \qquad (VII)$$

mit Magnesium, Lithium bzw. mit Alkyllithium, vorzugsweise n-Butyl-lithium. Hal bedeutet dabei Halogen, insbesondere Brom oder Chlor.

Verbindungen der Art (IV) mit n = 1 können auch aus Verbindungen der Art (VIII)

$$A\text{-}CH_3 \qquad (VIII)$$

derart hergestellt werden, daß man die am Aromaten stehende, aktivierte Methylgruppe mit starken Basen wie mit Alkyllithium, vorzugsweise n-Butyllithium, oder Lithiumamiden, vorzugsweise Lithiumdiisopropylamid deprotoniert. Aktivierte Methylgruppen sind beispielsweise in Verbindungen (VIII) mit A = 2-Pyridyl, 4-Pyridyl, 2-(4,4-Dimethyl-$\Delta^2$-oxazolino)-phenyl oder in 2- bzw. 4-Stellung durch -CO-$R_4$ substituiertes Phenyl, wobei $R_4$ substituiertes Amino bedeutet, enthalten.

Verbindungen der Art (IV) mit n = 0 können auch durch direkte Deprotonierung kernständiger, aktivierter Wasserstoffatome von Verbindungen (IX)

A-H

mittels Lithiumalkyl erhalten werden. Kernständige, aktivierte Wasserstoffatome stehen beispielsweise am Phenylrest in Nachbarstellung zu einer Alkoxy-, vorzugsweise Methoxygruppe oder auch in α-Stellung des Furyl- oder N-Alkyl-Pyrryl-Systems.

Die derart hergestellten Verbindungen der Formel (I) kann man als solche oder in Form ihrer pharmakologisch verwendbaren Säureadditionssalze gewinnen. Man kann sie aber auch nach den folgenden Varianten weiter zu anderen Verbindungen der Formel (I) umsetzen:

iii) Verbindungen, in denen A 2- oder 4-Brom-phenyl oder c2) bedeutet, werden mit Lithiumalkyl metalliert und in an sich bekannter Weise zu Verbindungen (I) mit A = c1), c3) bis c5), c10) weiter umgesetzt;

iv) Verbindungen, in denen A c2) bedeutet, werden in an sich bekannter Weise mit HJ zu Verbindungen c9) umgesetzt;

v) Verbindungen, in denen A c8) bedeutet, werden durch saure Hydrolyse zu dem entsprechenden Aldehyd umgesetzt.

Auch die nach den Varianten iii) bis v) entstandenen Verbindungen können als solche oder in Form ihrer pharmakologisch verwendbaren Säureadditionssalze gewonnen werden.

Die metallorganischen Reaktionen werden unter Ausschluß von Feuchtigkeit (absolutierte Lösungsmittel) und Luft-Sauerstoff (unter Inertgasatmosphäre, z.B. Stickstoff, Argon) durchgeführt.

Als inerte organische Lösungsmittel kommen in Frage Ether (z.B. Diethylether, Tetrahydrofuran, Dioxan, Dimethoxyethan) oder Kohlenwasserstoffe wie z.B. Hexan, Cyclohexan oder Benzol, vorzugsweise Diethylether, Tetrahydrofuran und Hexan bzw. deren Gemische.

In der Variante (i) erzeugt man zunächst aus dem entsprechenden Halogenid (VII) und fein verteiltem Magnesium entsprechend den Bedingungen der Grignard-Reaktion das Grignard-Reagenz (III) (z.B. K. Nützel, Methoden zur Herstellung und Umwandlung magnesiumorganischer Verbindungen, in: Houben-Weyl, Methoden der organischen Chemie, Band 13/2a, S. 53 ff).

Zur Erzeugung des Grignard-Reagenzes kann, falls notwendig, ein Katalysator verwendet werden, z.B. Jod oder 1,2-Dibromethan. Die Reaktionstemperatur liegt üblicherweise zwischen Raumtemperatur und dem Siedepunkt des Lösungsmittels oder des Lösungsmittelgemisches.

In Abhängigkeit von sterischen und elektronischen Eigenschaften der Halogenide (VII) kann die Reaktionsdauer zwischen 30 min und mehreren Stunden betragen.

Alternativ kann gemäß der Variante ii) auch eine lithiumorganische Reaktion zur Erzeugung der Verbindung (I) durchgeführt werden. Die Darstellung der Lithium-Zwischenstufe erfolgt nach Standardmethoden (z.B. H. Gilman, The Metalation Reaction With Organolithium Compounds, in: Organic Reactions, Vol. 8, S,. 258 ff oder H. R. Rodriguez, Heteroatom-Facilitated Lithiations, in; Organic Reactions, Vol. 26, S. 1 ff), beispielsweise Umsetzungen mit metallischem Lithium, Lithiumalkylen wie vorzugsweise n-Butyllithium oder Lithiumamiden wie vorzugsweise Lithiumdiisopropylamid.

Es ist bekannt, daß stickstoffhaltige Lewis-Basen die Reaktivität der Lithiumverbindungen steigern; daher werden, falls erforderlich, beispielsweise N,N,N',N'-Tetramethylethylendiamin (TMEDA) oder 1,4-Diazabicyclo-[2,2,2]-octan (DABCO) als Katalysatoren eingesetzt.

Die Reaktionstemperatur liegt üblicherweise zwischen −78°C und +40°C. In Abhängigkeit von den sterischen und elektronischen Eigenschaften des Halogenids (VII) kann die Reaktionsdauer zwischen 30 min und mehreren Stunden betragen.

Die nach der Verfahrensvariante iii) durch Umsetzung der Bromide oder Alkoxyverbindungen mit Lithiumalkyl, vorzugsweise n-Butyllithium, gebildeten Lithiumphenylderivate können in an sich bekannter Weise zu neuen Verbindungen (I) umgesetzt werden (z.B.: N.S. Narasimhan, R.S. Mali, Synthesis *1983*, S. 957 ff); beispielsweise erwähnt werden sollen Umsetzungen mit N,N-Dimethylformamid und Alkylhalogeniden.

Die Etherspaltung gemäß Variante iv) erfolgt nach an sich üblichen Methoden wie Behandlung mit Jodwasserstoffsäure, Bortribromid, Bortrichlorid oder Phosphorpentachlorid, vorzugsweise mit Jodwasserstoffsäure.

Die Umsetzungen können bei Normaldruck oder auch bei erhöhtem Druck durchgeführt werden, vorzugsweise bei Normaldruck.

Die Reaktionstemperaturen können je nach Verfahren variieren in einem Bereich von −78°C bis +200°C.

Die gegebenenfalls notwendige Reinigung der erhaltenen Verbindungen erfolgt in üblicher Weise, z.B. durch Säure-Base-Trennung oder chromatographische Verfahren.

Die erfindungsgemäß erhältlichen Säureadditionssalze erhält man durch an sich bekannte Umsetzung der basischen Verbindungen (I) mit Säuren, welche pharmakologisch verwendbare Salze bilden.

Die Verbindungen der Formel (I) und ihre pharmakologisch verwendbaren Säureadditionssalze zeichnen sich durch interessante pharmakologische Eigenschaften aus und zeigen insbesondere sauerstoffeinsparende, frequenzbeeinflussende und rhythmisierende Wirkungen am Herzen. Die neuen Verbindungen zeichnen sich aus durch eine gute Wirksamkeit und hohe Verträglichkeit.

So zeigen die neuen Verbindungen schon in geringen Dosen eine ausgeprügte Herzfrequenzsenkung und eine zusätzliche antiarrhythmische Wirkung. Darüber hinaus ist die unerwünschte negative Beeinflussung des Kontraktionsvermögens des Herzens äußerst gering. Das heißt, die Verbindungen weisen ein besonders günstiges Verhältnis von herzfrequenzsenkender bzw. die Refraktärzeit des Herzens verlängernder Wirkung zu negativ inotropen Nebenwirkungen auf und besitzen somit eine große therapeutische Breite.

Der Einfluß der Wirksubstanz auf den myokardialen Sauerstoffverbrauch wurde an narkotisierten Ratten untersucht und anhand der Methode von Neill [Neill, W.Z., H.H. Levine, R.J. Wagman, R. Gorlin, Circulation Research *12* (1963), 163] berechnet. Die hierzu erforderlichen Kreislaufmeßgrößen systolischer Blutdruck und Herzfrequenz wurden in der Versuchsanordnung nach Buschmann et al. [G. Buschmann, W. Schumacher, R. Budden und U.G. Kühl, J. Cardiovasc. Pharmacol. 2 (1980), 777 bis 795] ermittelt. Wie aus Tabelle A ersichtlich, reduzieren die Wirksubstanzen das Doppelprodukt aus Herzfrequenz und systolischem Blutdruck und führen damit zu einer Sauerstoffeinsparung am Herzen.

TABELLE A

Einfluß auf Herzfrequenz (FRQ), systolischen Blutdruck ($P_s$) und das Doppelprodukt (DP) narkotisierter Ratten

| Substanz | Dosis [μmol/kg i.v.] | FRQ [1/min] | $P_s$ [mmHg] | DP [mmHg/min × 100] | Änderung DP [Prozent] |
|---|---|---|---|---|---|
| Bsp. 2 | Vorwerte | 368 | 123 | 453 | – |
| Nr. 218 *) | 6,6 | 254 | 124 | 312 | –29 |
| Bsp. 2 | Vorwerte | 408 | 124 | 506 | – |
| Nr. 221 *) | 2,6 | 306 | 121 | 376 | –26 |

*) = eingesetzt als Dihydrochlorid.

Der Nachweis der antiarrhythmischen Wirkung der neuen Wirksubstanzen erfolgte experimentell durch Bestimmung der funktionellen Refraktärzeit des linken Herzvorhofes von weiblichen Albino Pirbright-white Meerschweinchen der Gewichtsklasse 300 bis 400 g mit Hilfe der gespaarten elektrischen Stimulation in Anlehnung an die Methode von Govier (W.C. Govier, J. Pharmacol. Exp. Ther. *148* (1) (1965), 100 bis 105). Sämtliche zur Zeit in der Therapie bereits eingesetzten Antiarrhythmika unterschiedlicher chemischer Struktur zeichnen sich durch eine Verlängerung der funktionellen Refraktärzeit aus. Zusätzlich gestattet die Methode die Erfassung von Substanzeinwirkungen auf die Kontraktionskraft des Herzmuskels. In der Tabelle B ist deshalb als FRP 125% diejenige Konzentration in μmol/l angegeben, bei der es 18 min nach Substanzapplikation zu einer Verlängerung der funktionellen Refraktärzeit auf 125% kommt bzw. als F 75% die entsprechende Konzentration, die eine Verminderung der Konzentrationskraft auf 75% des Ausgangswertes bewirkt.

Der direkte Einfluß der Wirksubstanz auf die Herzfrequenz (FRQ) wurde an spontan schlagenden, isolierten rechten Vorhöfen von weiblichen Albino Pirbright-white Meerschweinchen der Gewichtsklasse von 300 bis 400 g geprüft. In Tabelle B ist als FRQ 75% diejenige Konzentration in μmol/l angegeben, bei der es 20 min nach der Substanzgabe zu einer Abnahme der Frequenz auf 75% des Ausgangswertes kommt.

Angegeben ist weiterhin der Quotient F 75% / FRQ 75% aus kontraktionskraftmindernder und herzfrequenzsenkender Dosis. Dieser Quotient gibt Aufschluß über die therapeutische Breite der Substanzen in bezug auf die herzfrequenzsenkende Wirkung.

Aus der Tabelle B ergibt sich, daß die neuen substanzen keine nennenswerten unerwünschten negativ inotropen Effekte zeigen, jedoch bereits in sehr niedriger Konzentration eine herzfrequenzsenkende und eine antiarrhythmische Wirkung entfalten.

TABELLE B

Einfluss auf die Frequenz (FRQ) spontanschlagender rechter Meerscheinchenvorhöfe sowie auf die Kontraktionskraft (F) und die funktionelle Refraktärzeit (FRP) elektrisch gereizter linker Meerschweinchenvorhöfe

| Testsubstanz der Formel (I) | Effektive Konzentration (μmol/l) | | | Quotient F 75% / FRQ 75% |
|---|---|---|---|---|
| | FRQ 75% | F 75% | FRP 125% | |
| Bsp. 2 Nr. 218 *) | 2,06 | >46,4 | 6,24 | >22,6 |
| Bsp. 2 Nr. 221 *) | 0,99 | >46,4 | 2,17 | >46,8 |
| Pentylspartein **) | 7,5 | 22,1 | 9,1 | 2,9 |

*) = eingesetzt als Dihydrochlorid
**) = eingesetzt als Salz mit 2,3 mol L(+)-Weinsäure.

Tabelle B gibt auch die Ergebnisse der literaturbekannten Verbindung übersaures Pentylspartein-L(+)-tartrat wieder (EP-A-0 025 069). Der Vergleich zeigt deutlich die überragende Therapeutische Breite in bezug auf die herzfrequenzsenkende Wirkung der erfindungsgemäßen Verbindungen gegenüber der der bekannten Substanz.

Die überlegene Wirkung der erfindungsgemäßen Substanzen ist somit gekennzeichnet durch die Kombination von sauerstoffeinsparendem, rhythmisierendem und frequenzbeeinflussendem Effekt am Herzen.

Dieses Wirkprofil gestattet einen Einsatz bei der ischämischen Herzkrankheit, z.B. Angina pectoris und Myokardinfarkt, sowie bei lebensbedrohlichen Arrhythmien.

Die zu verwendenden Dosen varriieren naturgemäß je nach Art der verwendeten Substanz, der Applikationsart und des zu behandelnden Zustandes. Im allgemeinen werden jedoch befriedigende Ergebnisse in Tierversuchen mit Dosen von 0,01 bis 100 mg/kg Körpergewicht erhalten.

Die nachfolgende Beispiele sollen die Herstellung der neuen Verbindungen der Formel (I) näher erläutern, jedoch den Umfang der Erfindung in keiner Weise beschränken.

Die Strukturen der neuen Verbindungen wurden durch spektroskopische Untersuchungen, insbesondere durch Analyse der NMR-, Massen-, IR und/oder UV-Spektren gesichert.

Das 17-Hydroxyspartein wurde gemäß DE-OS 2 825 117 aus Spartein gewonnen.

Das 17-Dehydrosparteinperchlorat wurde nach M. Rink und K. Grabowski, Arch. Pharm. *289*, (1956) 695 aus 17-Hydroxyspartein hergestellt.

In den Beispielen und Tabellen werden folgende Abkürzungen verwendet:

LSM = Lösungsmittel, THF = Tetrahydrofuran, Et = Diethylether, T = Temperatur (°C), Kat = Katalysator, P = 17-Dehydrosparteinperchlorat, H = 17-Hydroxyspartein, DBE = 1,2-Dibromethan, WS = Weinsäure-Salz, HFu = Fumarsäure Salz.

*Beispiel 1:*

Herstellung der Verbindungen (III) bzw. (IV)

1a) Herstellung von Verbindungen (III)

0,1 mol Magnesiumspäne werden in 50 ml Lösungsmittel (absolutiert) vorgelegt. Gegebenenfalls wird Katalysator zugesetzt. Nach dem Zutropfen von 0,1 mol (VII) in 50 ml Lösungsmittel hält man den Ansatz bis zur Auflösung des Magnesiums an Rückfluß.

Der Reaktionsansatz wird als solcher für die Umsetzung in Beispiel 2 verwendet.

1b) Herstellung von Verbindungen (IV)

4,4 mmol n-Butyllithium (als 15%ige Lösung in Hexan) wird in 30 ml Lösungsmittel (absolutiert) bei −78°C gelöst. Nach dem langsamen Zutropfen von 4,5 mmol (VII) in 30 ml Lösungsmittel wird für weitere 30 min bei −78°C gerührt. Dieser Ansatz wird für die Umsetzung in Beispiel 3 verwendet.

1c) Herstellung von Verbindungen (IV)

Zur Lösung von 0,1 mol Lithium-Diisopropylamid — hergestellt in situ aus Diisopropylamin und n-Butyllithium — in 100 ml Lösungsmittel (absolutiert) werden bei entsprechender Temperatur 0,1 mol (VIII) eingetropft und weiter gerührt. Danach wird mit 200 ml Lösungsmittel verdünnt und der resultierende Ansatz für die Umsetzung im Beispiel 3 verwendet.

1d) Herstellung von (IV)

Zu 0,1 mol (IX) in 50 ml Lösungsmittel werden, gegebenenfalls in Anwesenheit etwa äquimolarer Menge an Katalysator, unter Kühlung (T1) 62,5 ml einer 1,6 molaren Lösung von n-Butyllithium in Hexan zugetropft. Zur Vervollständigung der Deprotonierung wird bei erhöhter Temperatur (T2) weitergerührt. Der resultierende Ansatz wird für die Umsetzung in Beispiel 3 verwendet, wobei vorher auf Temperatur T3 eingestellt wird.

Angaben zu speziellen Reaktionsbedingungen finden sich in Tabellen 1a) bis 1d).

*Beispiel 2:*

Herstellung von (I) gemäß Variante i)

In den aus Beispiel 1a) stammenden Ansatz wird 0,05 mol 17-Hydroxyspartein bzw. 17-Dehydrospartein-Perchlorat in 100 ml Lösungsmittel eingegeben und bis zur vollständigen Umsetzung am Rückfluß erwärmt. Nach vorsichtigem Ansäuern mit verdünnter Salzsäure und anschließender Säure/Base-Trennung und Chromatographie an neutralem Aluminiumoxid bzw. Kieselgel mit Äther/Hexan-Gemisch als Laufmittel wird die Verbindung (I) isoliert. In einer Variante wird diese zu ihrem Säureadditionssalz umgesetzt. Spezielle Reaktionsbedingungen und erhaltene Produkte werden in Tabelle 2 wiedergegeben.

*Beispiel 3:*

Herstellung von (I) gemäß Variante ii)

In den aus Beispiel 1b bzw. 1c) bzw. 1d) erhaltenen Ansatz wird die äquimolare Menge festes 17-Dehydrospartein-Perchlorat gegeben und unter langsamer Erwärmung auf Raumtemperatur bis zur vollständigen Umsetzung weitergerührt.

Nach Zugabe von verdünnter Säure oder Eiswasser wird wie in Beispiel 2 aufgearbeitet. Angaben zu speziellen Reaktionsbedingungen und zu erhaltenen Produkten werden in Tabelle 3 gegeben.

*Beispiel 4:*

Herstellung von (I) gemäß Variante iii)

0,013 mol Edukt werden in Lösungsmittel (absolutiert) gelöst, wobei gegebenenfalls Katalysator zugesetzt wird. Unter Kühlung setzt man tropfenweise 0,02 mol n-Butyllithium (als 15%ige Lösung in Hexan) zu und nach Rühren bei Raumtemperatur für 1 bis 6 Stunden erfolgt die Zugabe von 0,04 mol N,N-Dimethylformamid in 20 ml Lösungsmittel.

Nach Beendigung der Reaktion wird mit Wasser versetzt und wie in Beispiel 2 aufgearbeitet. Spezielle Reaktionsbedingungen und erhaltene Produkte werden in Tabelle 4 charakterisiert.

*Beispiel 5:*

Herstellung von (I) gemäß Variante iv)

Zu 0,017 mol Edukt in 14 ml Essigsäureanhydrid läßt man langsam 30 ml 57%ige Jodwasserstoffsäure tropfen. Der Ansatz wird 4 Stunden unter Rückfluß erhitzt. Man gibt das Reaktionsgemisch vorsichtig auf Eiswasser und arbeitet wie unter Beispiel 2 angegeben auf. Angaben zu Reaktionsbedingungen und zu erhaltenen Produkten werden in Tabelle 5 wiedergegeben.

*Beispiel 6:*

Herstellung von (I) gemäß Variante v)

0,02 mol Magnesium werden in 50 ml absolutem THF vorgelegt. Nach Aktivierung mit 0,2 ml 1,2-Dibromäthan erfolgt tropfenweise Zugabe von 0,02 mol 2-(3-Bromphenyl)-1,3-dioxolan in 10 ml absolutem THF. Nachdem der Ansatz 1 Stunde am Rückfluß erhitzt worden ist, verdünnt man mit 100 ml absolutem THF und versetzt mit 0,01 mol 17-Dehydrosparteinperchlorat und erhitzt erneut bis zur vollständigen Umsetzung.

Zur Freisetzung des Aldehyds wird mit verdünnter Salzsäure versetzt und 30 min bei Raumtemperatur gerührt. Nach Säure/Base-Trennung und anschliessender Filtration über Tonerde mit Äther/Hexan (1 : 1) als Laufmittel kann das Formylderivat als öl isoliert werden. Durch Umsetzung mit 3 Äquivalenten Weinsäure erhält man ein kristallines Tartrat (Schmelzpunkt: 126 bis 130°C).

Die folgenden Beispiele 7 bis 9 beschreiben pharmazeutische Zubereitungen enthaltend die erfindungsgemäßen Wirkstoffe sowie die Herstellung solcher pharmazeutischer Zubereitungen.

*Beispiel 7 - Tabletten:*

Zusammensetzung:

| | | |
|---|---|---|
| Wirkstoff (Beispiel 2 Nr. 218) | 20 | Teile |
| Maisstärke | 30 | Teile |
| Lactose | 55 | Teile |
| Kollidon 25$^R$ — | 5 | Teile |
| Magnesiumstearat | 2 | Teile |
| Hydriertes Rizinusöl | 1 | Teil |
| Total | 113 | Teile |

Herstellungsvorschrift:

Der Wirkstoff wird mit Maisstärke und feinpulvriger Lactose in einem Mischer vermischt. Die entstandene Mischung wird mit einer 20%igen Lösung von Polyvinylpyrrolidon (Kollidon 25, Fa. BASF) in Isopropanol durchfeuchtet.

Falls erforderlich, wird weiteres Isopropanol hinzugefügt. Das feuchte Granulat wird durch ein 2-mm Sieb passiert, bei 40°C auf Horden getrocknet und anschließend durch ein 1 mm-Sieb (Frewitt-Maschine) passiert. Nach dem Vermischen des Granulats mit Magnesiumstearat und hydriertem Rizinusöl werden damit Tabletten von 113 mg gepreßt, so daß jede Tablette 20 mg Wirkstoff enthält.

*Beispiel 8 - Kapseln*

Zusammensetzung:

| | | |
|---|---|---|
| Wirkstoff (Beispiel 2, Nr. 218) | 20 | Teile |
| Maisstärke | 20 | Teile |
| Lactose | 45 | Teile |
| Kollidon 25$^R$ | 3 | Teile |
| Magnesiumstearat | 1,5 | Teile |
| Aerosol 200$^R$ | 0,5 | Teile |
| Total | 90 | Teile |

Herstellungsvorschrift:

Der Wirkstoff wird mit Maisstärke und feinpulvriger Lactose in einem Mischer vermischt. Die entstandene Mischung wird mit einer 20%igen Lösung von Polyvinylpyrrolidon (Kollidon 25) in Isopropanol durchfeuchtet. Falls erforderlich, wird weiteres Isopropanol hinzugefügt. Das feuchte Granulat wird durch ein 1,6 mm-Sieb (Frewitt) passiert, bei 40°C auf Horden getrocknet und anschließend durch ein 1 mm-Sieb (Frewitt) passiert. Nach dem Vermischen des Granulats mit Magnesiumstearat und Kieselsäure-aerogel (Aerosil 200, Fa. Degussa) füllt man davon jeweils 90 mg mittels automatischer Kapselmaschine in Hartgelatinekapseln der Größe 4 ab, so daß jede Kapsel 20 mg Wirkstoff enthält.

*Beispiel 9 - Ampullen*

Zusammensetzung (pro Ampulle):

| | | | |
|---|---|---|---|
| Wirkstoff (Beispiel 2, Nr. 218) | | 5 | mg |
| Natriumchlorid | | 16 | mg |
| Wasser für injektionszwecke | ad | 2,0 | ml |

Herstellungsvorschrift:

Man löst Natriumchlorid in Wasser für Injektionszwecke auf, fügt den Wirkstoff hinzu und löst unter Rühren. Mit genügend Wasser für Injektionszwecke wird bis zum Endvolumen aufgefüllt. Der Ansatz wird durch ein Membranfilter 0,25 μ passiert. Man füllt in Braunglasampullen jeweils 2,15 ml ab und schmilzt sie zu. Bei 121°C wird 30 Minuten lang mit Dampf sterilisiert. 2 ml Injektionslösung enthalten 5 mg Wirkstoff.

TABELLE 1a

Herstellung von Verbindungen (III) aus (VII)

| Beispiel | LSM | Kat | A | n | Hal |
|---|---|---|---|---|---|
| 100 | THF | — | 2-Thienyl | 0 | Br |
| 101 | Et | Jod | Phenyl | 0 | Br |
| 102 | Et | Jod | 2-Methoxy-phenyl | 0 | Br |
| 103 | Et | Jod | 3-Methoxy-phenyl | 0 | Br |
| 104 | Et | Jod | 4-Methoxy-phenyl | 0 | Br |
| 105 | Et | Jod | 3,4-Methylendioxyphenyl | 0 | Br |
| 106 | Et | Jod | 3,4-Ethylendioxyphenyl | 0 | Br |
| 107 | Et | Jod | 2-Trifluormethylphenyl | 0 | Br |
| 108 | Et | Jod | 3-Trifluormethylphenyl | 0 | Br |
| 109 | Et | Jod | 4-Trifluormethylphenyl | 0 | Br |
| 110 | Et | Jod | 3-Chlorphenyl | 0 | Br |
| 111 | Et | Jod | 4-Chlorphenyl | 0 | Br |
| 112 | Et | Jod | 3-Fluorphenyl | 0 | Br |
| 113 | Et | Jod | 4-Fluorphenyl | 0 | Br |
| 114 | THF | $CH_3J$ | 3,4-Dimethoxyphenyl | 0 | Br |
| 115 | THF | $CCl_4$ | 3-Methylphenyl | 0 | Br |
| 116 | THF | — | Phenyl | 1 | Cl |
| 117 | Et | Jod | 2-Methoxyphenyl | 1 | Cl |
| 118 | Et | Jod | 3-Methoxyphenyl | 1 | Cl |
| 119 | Et | Jod | 4-Methoxyphenyl | 1 | Cl |
| 120 | THF | DBE | 3,4,5-Trimethoxyphenyl | 1 | Cl |
| 121 | Et | Jod | 2-Chlorphenyl | 1 | Cl |
| 122 | Et | Jod | 3-Chlorphenyl | 1 | Cl |
| 123 | Et | — | 4-Chlorphenyl | 1 | Br |
| 124 | Et | — | 3-Trifluormethylphenyl | 1 | Cl |
| 125 | Et | — | 2-Fluorphenyl | 1 | Br |
| 126 | Et | — | 3-Fluorphenyl | 1 | Br |
| 127 | Et | — | 4-Fluorphenyl | 1 | Br |
| 128 | Et | — | 2-Bromphenyl | 1 | Br |
| 129 | Et | DBE | 3-Bromphenyl | 1 | Br |
| 130 | Et | DBE | 4-Bromphenyl | 1 | Br |
| 131 | Et | — | 3,4-Dichlorphenyl | 1 | Br |
| 132 | Et | — | 2,6-Dichlorphenyl | 1 | Br |
| 133 | Et | — | 2-Methylphenyl | 1 | Br |
| 134 | Et | — | 3-Methylphenyl | 1 | Br |
| 135 | Et | — | 4-Methylphenyl | 1 | Br |
| 136 | Et | — | 2,4-Dimethylphenyl | 1 | Cl |
| 137 | Et | — | 3,5-Dichlorphenyl | 1 | Cl |
| 138 | Et | — | 2,6-Difluorphenyl | 1 | Br |
| 139 | Et | — | 2-Fluor-3-methylphenyl | 1 | Br |
| 140 | Et | — | 2-Chlor-6-fluorphenyl | 1 | Cl |

TABELLE 1b - Herstellung von Verbindungen (IV) aus (VII)

| Beispiel | LSM | A | n | Hal |
|---|---|---|---|---|
| 150 | THF | 2-Pyridyl | 0 | Br |
| 151 | Et | 4-Bromphenyl | 0 | Br |

TABELLE 1c - Herstellung von Verbindungen (IV) aus (VIII)

| Beispiel | LSM | T | A | n |
|---|---|---|---|---|
| 170 | THF | −78 | 4-Pyridyl | 1 |
| 171 *) | THF | −78 | 2-Pyridyl | 1 |
| 172 **) | THF | 0 | 2,3-dimethoxyphenyl | 1 |
| 173 | THF | 0 | 4-N,N-Diisopropylbenzamid | 1 |
| 174 *) | Et | 0 | 2-(4,4-Dimethyl-$\Delta^2$-oxazolino)-phenyl | 1 |

Bemerkung: *) = n-Butyllithium statt Diisopropylamid **) = unter Zusatz von TMEDA

TABELLE 1d - Herstellung von Verbindungen (IV) aus (IX)

| Beispiel | LSM | Kat | $T_1$ | $T_2$ | $T_3$ | A | n |
|---|---|---|---|---|---|---|---|
| 190 | THF | — | −25 | −15 | 0 | 2-Furyl | 0 |
| 191 | THF | TMEDA | 0 | +40 | −30 | 2-(N-Methyl)pyrryl | 0 |

TABELLE 2 - Herstellung von Verbindungen (I) gemäß Variante i)

| Beispiel | Spartein-derivat | LSM | A | n | isolierte Form | Smp |
|---|---|---|---|---|---|---|
| 200 | P | THF | 2-Thienyl | 0 | Base | 84 |
| 201 | H | Et | Phenyl | 0 | Base | 105-107 |
| 202 | P | Et | 2-Methoxyphenyl | 0 | 2 HCl | 220 |
| 203 | P | Et | 3-Methoxyphenyl | 0 | 2 HCl | 208 |
| 204 | P | Et | 4-Methoxyphenyl | 0 | 2 HCl | 238 |
| 205 | P | Et | 3,4-Methylendioxyphenyl | 0 | 2,33 WS | 145 |
| 206 | P | Et | 3,4-Ethylendioxyphenyl | 0 | 1,9 HFu | 187 |
| 207 | P | Et | 2-Trifluormethylphenyl | 0 | Base | 130 |
| 208 | H | Et | 3-Trifluormethylphenyl | 0 | 2 HCl | amorph |
| 209 | H | Et | 4-Trifluormethylphenyl | 0 | 2 HCl | amorph |
| 210 | H | Et | 3-Chlorphenyl | 0 | 3 WS | amorph |
| 211 | H | Et | 4-Chlorphenyl | 0 | 3,4 WS | amorph |
| 212 | H | Et | 3-Fluorphenyl | 0 | 3 WS | amorph |
| 213 | H | Et | 4-Fluorphenyl | 0 | 3,6 WS | amorph |
| 214 | P | THF | 3,4-Dimethoxyphenyl | 0 | Base | 117 |
| 215 | P | THF | 3-Methylphenyl | 0 | 2,3 HFu | amorph |
| 216 | P | THF | Phenyl | 1 | Base | 68-71 |
| 217 | P | Et | 2-Methoxyphenyl | 1 | Base | 59 |
| 218 | P | Et | 3-Methoxyphenyl | 1 | Base | 51-52 |
| 219 | H | Et | 4-Methoxyphenyl | 1 | Base | 100 |
| 220 | P | THF | 3,4,5-Trimethoxyphenyl | 1 | 2,8 WS | 103 |

TABELLE 2 - Herstellung von Verbindungen (I) gemäß Variante i) (Fortsetzung)

| Beispiel | Spartein-derivat | LSM | A | n | isolierte Form | Smp |
|---|---|---|---|---|---|---|
| 221 | H | Et | 2-Chlorphenyl | 1 | Base | 115 |
| 222 | H | Et | 3-Chlorphenyl | 1 | Base | 76 |
| 223 | P | Et | 4-Chlorphenyl | 1 | Base | 120 |
| 224 | H | Et | 3-Trifluormethylphenyl | 1 | 2,8 HFu | amorph |
| 225 | P | THF | 2-Fluorphenyl | 1 | 2,25 WS | 126 |
| 226 | P | THF | 3-Fluorphenyl | 1 | 2,15 WS | 138 |
| 227 | P | THF | 4-Fluorphenyl | 1 | Base | 74 |
| 228 | P | THF | 2-Bromphenyl | 1 | Base | 131 |
| 229 | P | THF | 3-Bromphenyl | 1 | 2 WS | 138-140 |
| 230 | P | THF | 4-Bromphenyl | 1 | Base | 142 |
| 231 | P | THF | 3,4-Dichlorphenyl | 1 | Base | 80 |
| 232 | P | THF | 2,6-Dichlorphenyl | 1 | 2,1 WS | 143 |
| 233 | P | THF | 2-Methylphenyl | 1 | 2,1 WS | 140 |
| 234 | P | THF | 3-Methylphenyl | 1 | Base | 57 |
| 235 | P | THF | 4-Methylphenyl | 1 | Base | 58 |
| 236 | P | THF | 2,4-Dimethylphenyl | 1 | Base | 158-161 |
| 237 | P | THF | 3,5-Dichlorphenyl | 1 | 2,1 WS | 130 |
| 238 | P | THF | 2,6-Difluorphenyl | 1 | 2,2 WS | 135 |
| 239 | P | THF | 2-Fluor-3-methylphenyl | 1 | 2,1 WS | 139 |
| 240 | P | THF | 2-Chlor-6-fluorphenyl | 1 | 2,2 WS | 131 |

TABELLE 3 - Herstellung von Verbindungen (I) gemäß Variante ii)

| Beispiel | Ansatz | n | A | isolierte Form | Smp |
|---|---|---|---|---|---|
| 350 | 1b | 0 | 2-Pyridyl | 2 WS | 135 |
| 351 | 1b | 0 | 4-Bromphenyl | 2,3 WS | 150 |
| 370 | 1c | 1 | 4-Pyridyl | 3 WS | 120 |
| 371 | 1c | 1 | 2-Pyridyl | Base | 128 |
| 372 | 1c | 1 | 2,3-Dimethoxyphenyl | 2 HCl | amorph |
| 373 | 1c | 1 | 4-N,N-diisopropylamino-carbonylphenyl | 2,5 WS | 145-148 |
| 374 | 1c | 1 | 2-(4,4-Dimethyl-$\Delta^2$-oxazolino)-phenyl | Base | 112-119 |
| 390 | 1d | 0 | 2-Furyl | 2,5 WS | 113 |
| 391 | 1d | 0 | 2-(N-Methyl)-pyrryl | Base | 118 |

TABELLE 4 - Herstellung von Verbindungen (I) gemäß Variante iii)

| Beispiel | Edukt | LSM | Kat | T | A | n | isolierte Form | Smp |
|---|---|---|---|---|---|---|---|---|
| 400 | 218 | THF | TMEDA | 0 | 3-Methoxy--4-formyl-phenyl | 1 | 2,3 WS | 117-120 |
| 401 | 351 | THF | — | −30 | 4-Formyl-phenyl | 0 | Base | 106 |

TABELLE 5 - Herstellung von Verbindungen (I) gemäß Variante iv)

| Beispiel | Edukt | A | n | isolierte Form | Smp |
|---|---|---|---|---|---|
| 500 | 204 | 4-Hydroxyphenyl | 0 | 2 HCl | amorph |
| 501 | 218 | 3-Hydroxyphenyl | 1 | Base | 74-76 |

**Patentansprüche**

1. Aromatische Verbindungen der allgemeinen Formel (I)

$$S\text{-}(CH_2)_n\text{-}A \qquad (I)$$

in der
S den 17-Spartein-Rest
n 0 oder 1
und A

a1) 2-Furyl, 2-Thienyl, 2-(N-$C_1$-$C_4$-Alkyl)-pyrryl für n = 0 oder
a2) 3-Furyl, 3-Thienyl für n = 1 oder
b) Pyridyl, oder
c) substituiertes Phenyl der Formel (II)

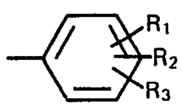
(II)

bedeutet, in dem unabhängig voneinander einer oder zwei der Reste $R_1$ bis $R_3$ Wasserstoff und einer oder mehrere der Reste $R_1$ bis $R_2$ unabhängig voneinander

C1) Alkyl mit 1-4 Kohlenstoffatomen,
C2) Alkoxy mit 1-4 Kohlenstoffatomen,
C3) Fluor,
C4) Chlor,
C5) Brom,
C6) Trifluormethyl,
C7) zwei benachbarte Reste gemeinsam Alkylendioxy mit 1-2 Kohlenstoffatomen,
C8) $\text{-}CH\text{-}O\text{-}(CH_2)_2\text{-}O,$ (ring closure between CH and O)
C9) Hydroxy,
C10) -Co-$R_4$

sind, wobei $R_4$ Wasserstoff, Alkoxy mit 1-4 Kohlenstoffatomen, Hydroxy oder gegebenenfalls durch Alkylgruppen mit 1-4 Kohlenstoffatomen substituiertes Amino ist,
sowie deren pharmakologisch verwendbare Säureadditionssalze.

2. Verbindungen gemäß Anspruch 1, dadurch gekennzeichnet, daß zwei der Reste $R_1$ bis $R_3$ Wasserstoff sind.

3. Verbindungen gemäß Anspruch 1, dadurch gekennzeichnet, daß einer der Reste $R_1$ bis $R_3$ Wasserstoff ist.

4. Verbindungen gemäß einem der Ansprüche 1 bis 3, dadurch gekennzeichnet, daß n = 1 ist.

5. Verbindungen gemäß Anspruch 2, dadurch gekennzeichnet, daß n = 1 ist.

6. Verbindungen gemäß Anspruch 5, dadurch gekennzeichnet, daß A Methoxyphenyl, insbesondere 3-Methoxyphenyl oder Chlorphenyl, insbesondere 2-chlorphenyl ist.

7. Arzneimittel enthaltend eine pharmakologische wirksame Menge einer Verbindung gemäß einem der vorangehenden Ansprüche oder eine Verbindung der Formel (I), in der A zusätzlich unsubstituiertes Phenyl bedeutet, oder pharmakologisch verwendbare Säureadditionssalze solcher Verbindungen und übliche pharmazeutische Hilfs- und/oder Trägerstoffe.

8. 17-Benzylspartein, 17-Phenylspartein und deren pharmakologisch verwendbare Säureadditionssalze zur Anwendung als Pharmazeutica.

9. Verfahren zur Herstellung von Verbindungen gemäß Anspruch 1, dadurch gekennzeichnet, daß man

i) 17-Hydroxyspartein oder 17-Dehydrospartein-Salz mit einer Grignard-Verbindung der Formel (III)

$$A\text{-}(CH_2)_n\text{-}MgHal \qquad (III)$$

oder

ii) 17-Dehydroxyspartein-Salz mit einer metallorganischen Verbindung der Formel (IV)

$$A\text{-}(CH_2)_n\text{-}Li \qquad (IV)$$

umsetzt, wobei n und A die in Anspruch 1 angegebene Bedeutung besitzen und Hal Halogen bedeutet und die resultierende Verbindungen entweder als solche oder in Form ihrer pharmakologisch verwendbaren Säureadditionssalze gewinnt oder sie folgendermaßen weiter zu anderen Verbindungen der Formel (I) umsetzt:

iii) Verbindungen in denen A 2- oder 4-Brom-phenyl oder c2) bedeutet, werden mit Lithiumalkyl metalliert und in an sich bekannter Weise zu Verbindungen (I) mit A = c1), c3) bis c5), c10) weiter umgesetzt

iv) Verbindungen, in denen A c2) bedeutet, werden in an sich bekannter Weise mit HJ zu Verbindungen, in denen A c9) bedeutet, umgesetzt

v) Verbindungen in denen A c8) bedeutet, werden durch saure Hydrolyse zu dem entsprechenden Aldehyd umgesetzt
und die derart erhaltenen Verbindungen als solche oder in Form ihrer pharmakologisch verwendbaren Säureadditionssalze gewinnt.

**Claims**

1. Aromatic compounds of the general Formula (I)

$$S\text{-}(CH_2)_n\text{-}A \qquad (I)$$

in which

S denotes the 17-spartein radical
n denotes 0 or 1
and A denotes

a1) 2-furyl, 2-thienyl, 2-(N-$C_1$-$C_4$-alkyl)-pyrryl for n = 0 or
a2) 3-furyl, 3-thienyl for n = 1 or
b) pyridyl or,
c) substituted phenyl of Formula (II)

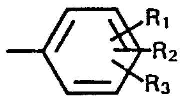 (II)

in which independently of each other one or two of the radicals $R_1$ to $R_3$ are hydrogen and one or more of the radicals $R_1$ to $R_3$ independently of each are

C1) alkyl with 1-4 carbon atoms
C2) alkoxy with 1-4 carbon atoms
C3) fluorine
C4) chlorine
C5) bromine
C6) trifluoromethyl
C7) two adjacent radicals together alkylene dioxy with 1-2 carbon atoms
C8) $-CH-O-(CH_2)_2-O$
C9) hydroxy
C10) $-CO-R_4$

in which $R_4$ is hydrogen, alkoxy with 1-4 carbon atoms, hydroxy or amino optionally substituted by alkyl with 1-4 carbon atoms and their pharmacologically usable acid addition salts.

2. Compounds according to Claim 1, characterized in that two of the radicals $R_1$ to $R_3$ are hydrogen.

3. Compounds according to Claim 1, characterized in that one of the radicals $R_1$ to $R_3$ is hydrogen.

4. Compounds according to one of Claims 1 to 3, characterized in that n = 1.

5. Compounds according to Claim 2, characterized in that n = 1.

6. Compounds according to Claim 5, characterized in that A is methoxyphenyl, in particular 3-methoxyphenyl or chlorophenyl, in particular 2-chlorophenyl.

7. Medicament containing a pharmacologically effective amount of a compound according to one of the preceding claims or a compound fo Formula (I), in which A denotes additionally unsubstituted phenyl, or a pharmacologically usable acid addition salt of such compounds and conventional auxiliary and/or carrier substances.

8. 17-Benzylspartein, 17-phenylspartein and their pharmacologically usable acid addition salts for use as pharmaceuticals.

9. Method for the preparation of compounds according to Claim 1, characterized in that

i) 17-hydroxyspartein or 17-dehydrospartein salt is reacted with a Grignard compound of Formula (III)

$$A\text{-}(CH_2)_n\text{-}MgHal \qquad (III)$$

or

ii) 17-dehydrospartein salt is reacted with an organometallic compound of Formula (IV)

$$A\text{-}(CH_2)_n\text{-}Li \qquad (IV)$$

in which n and A have the meanings given in Claim 1 and Hal denotes halogen,
and the resulting compounds are either obtained as such or in the form of their pharmacologically usable acid addition salts or they are further reacted as follows to other compounds of Formula (I):

iii) compounds in which A denotes 2- or 4-bromophenyl or c2), are metalated with lithium alkyl in a manner known *per se* are further reacted to compounds (I) with A = c1), c3) to c5), c10)

iv) compounds in which A denotes c2) are reacted in a manner known *per se* with HI to compounds in which A denotes c9),

v) compounds in which A denotes c8) are reacted by acid hydrolysis to the corresponding aldehyde

and the resulting compounds are obtained as such or in the form of their pharmacologically usable acid addition salts.

**Revendications**

1. Composés aromatiques de formule générale (I)

$$S\text{-}(CH_2)_n\text{-}A \qquad (I)$$

dans laquelle
S représente le reste 17-spartéine;
n vaut 0 ou 1;
et A représente

a1) un groupe 2-furyle, 2-thiényle, 2-(N-alkyl en $C_1$ à $C_4$)-pyrryle pour n nul ou bien
a2) un groupe 3-furryle ou 3-thiényle pour n valant 1, ou bien
b) un groupe pyridyle ou bien
c) un groupe phényle substitué, de formule (II)

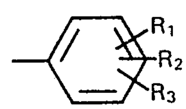 (II)

dans laquelle, indépendamment les uns des autres, un ou deux des restes $R_1$ à $R_3$ représentent un atome d'hydrogène et un ou plusieurs des restes $R_1$ à $R_3$ représentent, indépendamment l'un de l'autre:

C1) un groupe alkyle ayant 1 à 4 atomes de carbone,
C2) un groupe alcoxy ayant 1 à 4 atomes de carbone,
C3) un atome de fluor;
C4) un atome de chlore;
C5) un atome de brome;
C6) un groupe trifluorométhyle;
C7) deux restes voisins forment ensemble un groupe alkylènedioxy ayant 1 ou 2 atomes de carbone,
C8) $-CH-O-(CH_2)_2-O$,
C9) un groupe hydroxyle,
C10) un groupe $-CO-R_4$

où $R_4$ représente un atome d'hydrogène, un groupe alcoxy ayant 1 à 4 atomes de carbone, hydroxyle ou amino, lequel est éventuellement substitué par des groupes alkyles ayant 1 à 4 atomes de carbone, ainsi que leurs sels d'addition d'acides utilisables pharmacologiquement.

2. Composés selon la revendication 1, caractérisés en ce que deux des restes $R_1$ à $R_3$ représentent chacun un atome d'hydrogène.

3. composés selon la revendication 1, caractérisés en ce que l'un des restes $R_1$ à $R_3$ est un atome d'hydrogène.

4. Composés selon l'une des revendications 1 à 3, caractérisés en ce que n vaut 1.

5. Composés selon la revendication 2, caractérisés en ce que n vaut 1.

6. Composés selon la revendication 5, caractérisés en ce que A représente un groupe méthoxyphényle, en particulier un groupe 3-méthoxyphényle, ou un groupe chlorophényle, en particulier un groupe 2-chlorophényle.

7. Médicament contenant une quantité, pharmacologiquement active, d'un composé selon l'une des revendications précédentes ou d'un composé de formule (I), dans lequel A peut représenter en outre un groupe phényle non substitué, ou des sels d'addition d'acides, pharmacologiquement utilisables, de tels composés, et des adjuvants et/ou des véhicules, supports ou excipients pharmaceutiques usuels.

8. La 17-benzylspartéine, la 17-phénylspartéine et leurs sels d'addition d'acides, pharmacologiquement utilisables, pour servir de produits pharmaceutiques.

9. Procédé de préparation de composés selon la revendication 1, caractérisé en ce que l'on fait réagir

i) la 17-hydroxyspartéine ou un sel de 17-déshydrospartéine, avec un composé de Grignard répondant à la formule (III)

$$A\text{-}(CH_2)_n\text{-}Mg\ Hal \qquad (III)$$

ou bien

ii) un sel de 17-déshydrospartéine avec un composé organo-métallique de formule (IV)

$$A\text{-}(CH_2)_n\text{-}Li \qquad (IV)$$

les symboles m et A ayant le sens indiqué à la revendication 1 et Hal représentant un atome d'halogène,

et l'on obtient les composés résultants, tels quels ou sous forme de leurs sels d'addition d'acides pharmacologiquement utilisables, ou bien on les fait encore réagir, comme suit, pour obtenir d'autres composés de formule (I);

iii) dans des composés dans lesquels A représente un groupe 2- ou 4-bromophényle ou c2), on introduit, à l'aide d'un alkyl-lithium, un atome de métal et l'on fait encore réagir, de façon connue en soi, pour obtenir des composés (I) dans lesquels A représente c1), c3) à c5) ou c10);

iv) on fait réagir des composés, dans lesquels A représente c2), en opérant de façon connue en soi, avec HI pour obtenir des composés dans lesquels A représente c9);

v) on fait réagir des composés, dans lesquels A représente c8), par hydrolyse acide pour obtenir l'aldéhyde correspondant,

et l'on récupère tels quels les composés ainsi obtenus ou bien on les récupère sous forme de leurs sels d'addition d'acides, pharmacologiquement utilisables.